# FASCICULE DE BREVET EUROPEEN

(11) **EP 2 785 675 B1**
(45) Date de publication et mention de la délivrance du brevet: **18.11.2015**
(21) Numéro de dépôt: 12806601.6
(22) Date de dépôt: 29.11.2012
(51) Int. Cl.: C07C 49/483, C11B 9/00, C07C 49/453

(54) **5,5-DIMÉTHYL-2-PROPYL-HEXAHYDRO-2,4A-MÉTHANO-NAPHTHALÈN-1-ONE EN TANT QU'AGENT FRAGRANT**
5,5-DIMETHYL-2-PROPYL-HEXAHYDRO-2,4A-METHANONAPHTHALEN-1-ON ALS DUFTSTOFF
5,5-DIMETHYL-2-PROPYL-HEXAHYDRO-2,4A-METHANONAPHTHALENE-1-ONE AS A FRAGRANCE AGENT

(30) Priorité: 02.12.2011 FR 1161092
(43) Date de publication de la demande: 08.10.2014
(73) Titulaire: V. Mane Fils, 06620 Le Bar-sur-Loup (FR)
(72) Inventeur: JAUNKY, Piotr, 06740 Chateauneuf (FR); CHANOT, Jean-Jacques, 06530 Speracedes (FR); MANE, Jean, 06130 Magagnosc (FR)
(74) Mandataire: Richaud, Fabien
(86) Numéro de dépôt international: PCT/FR2012/052758
(87) Numéro de publication internationale: WO 2013/079876

(56) Documents cités:
- EP-A1- 0 029 259
- US-A- 4 250 338

## Description

La présente invention concerne de nouveaux composés cétoniques, ainsi que leurs utilisations dans l'industrie de la chimie, et en particulier en parfumerie, cosmétique, et dans l'industrie des détergents, lesdits composés présentant une fragrance et une rémanence particulières.

L'industrie des parfums est toujours à la recherche de composés organoleptiques nouveaux, présentant un pouvoir olfactif intense, tout en ayant des coûts de production les plus limités possibles. Plus particulièrement, des composés exhibant des notes ambrées pures, linéaires et dominantes sont rares et difficiles à obtenir. En effet, la plupart des composés odorants possédant des notes ambrées, possèdent également des notes tabac, boisées-pin (Angew. Chem. Int. Ed. 2000, 39, 2980-3010), et il est par conséquent difficile d'obtenir des notes ambrées dominantes pures, et présentant une forte substantivité.

Après maintes recherches sur les éthers, les cétones et les cétals cycliques, composés bien connus pour présenter des notes similaires à des notes ambrées (Angew. Chem. Int. Ed. 2000, 39, 2980-3010), la Demanderesse a découvert un composé de formule générale (I) suivante, 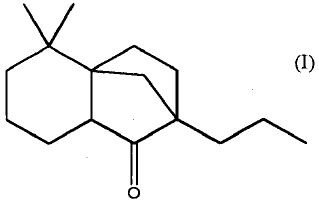 dénommé 5,5-Diméthyl-2-propyl-hexahydro-2,4a-méthano-naphthalèn-1-one, ledit composé exhibant des notes dominantes ambrées pures, linéaires, et surtout présentant une forte rémanence et donc une forte substantivité. Outre les qualités organoleptiques citées plus tôt, le composé selon la présente invention présente l'avantage d'être obtenu facilement à l'aide d'un procédé de synthèse simple et économique.

Des structures analogues, ou même des formules de Markush intégrant le composé de formule (I) de la présente invention, ont été divulguées dans l'art antérieur, notamment dans les brevets EP 0029259 et US 4250338. Mais le composé de formule (I) de la présente invention n'a jamais été décrit en lui-même ou synthétisé auparavant. Dans ces deux brevets EP 0029259 et US 4250338, des composés de formule (II) suivante sont divulgués, formule dans laquelle R1-R3 représentent un hydrogène, ou un groupement comprenant de 1 à 3 carbones.

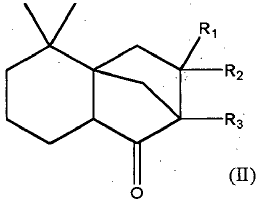

Les composés décrits dans ces deux brevets développent des odeurs multi-facettes, mais marquées par une forte odeur boisée, toujours accompagnée par des notes camphrées indésirables. De plus, dans les brevets EP 0029259 et US 4250338, il est décrit que pour développer des notes ambrées, les composés cétoniques divulgués devaient subir d'autres étapes de procédé afin d'obtenir les dérivés alcool, acétate ou méthyl ether correspondants, ce qui entraine un procédé de synthèse plus long et donc des coûts plus élevés. Au final, lesdits dérivés possèdent néanmoins toujours des odeurs multi-facettes.

Ainsi, un premier objet de la demande se rapporte à un composé de formule générale (I) suivante : 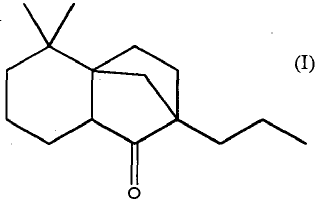 ainsi que ses stéréoisomères.

Un second objet de la présente demande se rapporte à une composition comprenant au moins un composé de formule (I) ou l'un de ses stéréoisomères ou un mélange de ceux-ci.

Enfin, un dernier objet de la présente demande concerne l'utilisation d'au moins un composé de formule (I) ou l'un de ses stéréoisomères, en tant qu'agent ou composé fragrant.

La figure 1 se rapporte à un procédé de synthèse des composés de formule (I) et de ses stéréoisomères.

Le composé de formule (I) selon la présente invention, présente l'avantage d'exhiber une odeur ambrée très dominante, linéaire, pure et très rémanente. Le composé selon l'invention se caractérise, d'une part, par le fait qu'il ne dégage aucune note indésirable camphrée et, d'autre part, par une très forte substantivité. En effet, le composé de la présente invention se caractérise par une rémanence bien plus importante que celle des composés similaires de l'art antérieur ayant des notes dominantes ambrées : ladite rémanence est d'environ deux semaines pour le composé de formule (I), comparée à celle des autres molécules de l'art antérieur pour lesquelles la rémanence est de moins d'une semaine.

Plus particulièrement, le composé de formule (I) est caractérisé en ce que le groupement décaline est dans une configuration cis (Ia) ou (Ib).

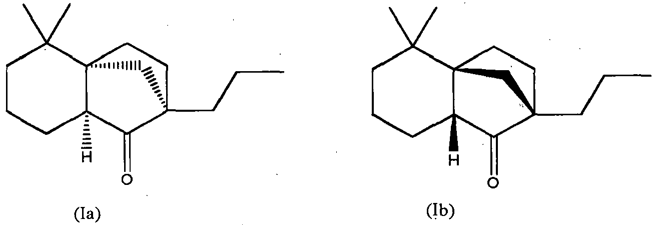

Dans un autre mode de réalisation, le groupement décaline du composé de formule (I) est dans une configuration trans (Ic) ou (Id).

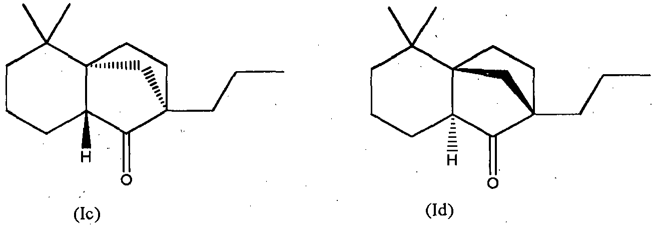

Par décaline on entend un composé organique bicyclique, aussi appelé décahydronaphthalène.

Dans un mode de réalisation préféré, le composé selon l'invention est de formule (Ia) ou (Ib). Préférentiellement, le composé selon l'invention est présent sous forme d'un mélange racémique des composés cis-décaline (Ia) et (Ib).

Le composé de formule (I) selon la présente invention peut être obtenu par l'intermédiaire d'un procédé de synthèse simple et économique, contrairement à tous les procédés longs et coûteux permettant d'obtenir des composés à notes ambrées. Par exemple, un mélange racémique des composés (Ia) et (Ib) (puis (Ic) et (Id)) est obtenu simplement et directement par une réaction de Diels-Alder faisant réagir du myrcène et du 2-méthylènepentanal, suivie d'un traitement acide. La figure 1 ci-dessous reprend les principales étapes de cette synthèse.

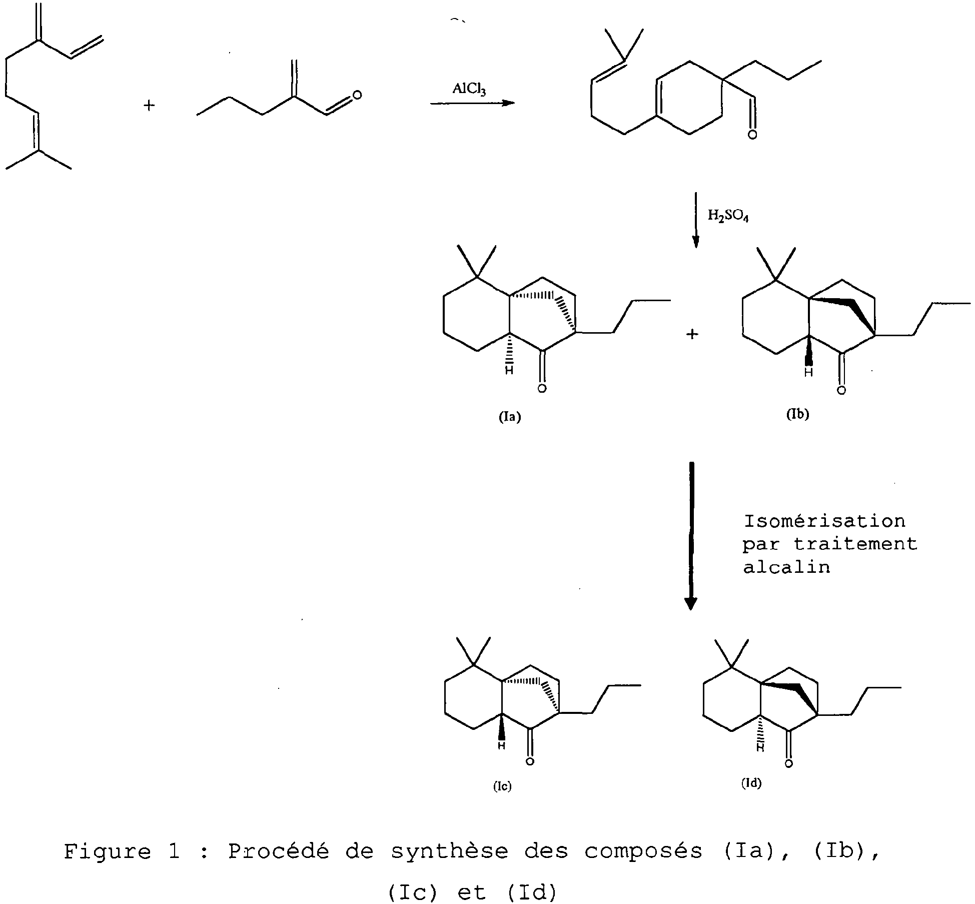

Une fois les composés cis-décaline (Ia) et (Ib) obtenus, ceux-ci peuvent être transformés en dérivés trans-décaline (Ic) et (Id) par des méthodes simples connues de l'homme du métier (par exemple par un traitement alcalin).
Dans les deux cas, les composés synthétisés présentent des notes ambrées linéaires franches.

Un second objet de la présente invention concerne une composition comprenant au moins un composé de formule générale (I), (Ia), (Ib), (Ic) ou (Id) sous la forme d'un stéréoisomère ou d'un mélange de stéréoisomères ou d'un mélange racémique.

Selon un mode de réalisation particulier, la composition est caractérisée en ce qu'elle comprend en outre au moins une autre substance odorante.

La quantité efficace des composés de formule (I) selon l'invention incorporée dans la composition variera selon la nature de la composition, l'effet odorant souhaité, et la nature des autres composés odorants ou non éventuellement présents, et pourra être aisément déterminée par l'homme du métier, sachant qu'elle peut varier dans une plage très étendue, de 0,1 à 99% en poids, en particulier de 0,1 à 50% en poids, notamment de 0,1 à 30% en poids par rapport au poids total de la composition.

L'invention concerne également en particulier une composition cosmétique, notamment crème pour le visage et le corps, poudre de talc, huile pour cheveux ou pour le corps, shampoing, lotion capillaire, sel de bain, huile de bain, gel de douche, gel de bain, savon de toilette, antitranspirant corporel, désodorisant corporel, lotions, crème de rasage, savon de rasage, crème, dentifrice, bain de bouche, pommade comprenant au moins un composé de formule (I), ou au moins une composition comprenant au moins un composé de formule (I).

L'invention concerne encore un produit d'entretien, notamment assouplissant, détergent, lessive, désodorisant d'ambiance, comprenant au moins un composé de formule (I) ou au moins une composition comprenant au moins un composé de formule (I).

Le ou les composés selon l'invention peuvent être utilisés, seuls ou en combinaison, tels quels ou être incorporés dans ou sur un matériau support inerte ou qui peut contenir d'autres ingrédients actifs de la composition finie. Une grande variété de matériaux supports peut être employée incluant, par exemple, les solvants polaires, les huiles, les graisses, les solides finement divisés, les cyclodextrines, les maltodextrines, les gommes, les résines et n'importe quel autre matériau support connu pour de telles compositions.

L'invention a pour dernier objet l'utilisation d'au moins un composé de formule (I) selon l'invention comme agent ou composé fragrant, comme agent de masquage d'odeur ou comme agent de neutralisation d'odeur. Le terme «fragrant», est utilisé ici pour désigner tout composé organoleptique stimulant de façon plaisante l'odorat. Par le terme «agent de masquage» ou «masquant» on entend réduire ou éliminer la perception d'une mauvaise odeur générée par une ou plusieurs molécules entrant dans la composition d'un produit.

En outre, ledit composé peut être utilisé seul ou en combinaison avec au moins un autre ingrédient aromatisant ou parfumant, et/ou au moins un solvant, et/ou au moins un adjuvant. Le ou les agents odorants supplémentaires peuvent être des composés de formule (I) ou d'autres agents odorants connus de l'homme du métier qui sera à même de choisir en fonction de l'effet recherché.

De manière générale, les composés selon l'invention seront utilisés dans le domaine de la parfumerie. On entend par « parfumerie » non seulement la parfumerie au sens habituel du terme, mais également les autres domaines dans lesquels l'odeur des produits est importante. Il peut s'agir de compositions de parfumerie au sens habituel du terme, telles que bases et concentrés parfumant, eaux de Cologne, eaux de toilette, parfums et produits similaires ; de compositions topiques - en particulier cosmétiques - telles que crèmes pour le visage et le corps, poudres de talc, huiles pour cheveux, shampoings, lotions capillaires, sels et huiles de bain, gels de douche et de bain, savons de toilette, anti-transpirants et désodorisants corporels, lotions et crèmes de rasage, savons, crèmes, dentifrices, bains de bouche, pommades, et produits similaires ; et de produits d'entretien, tels qu'assouplissants, détergents, lessives, désodorisants d'ambiance, et produits similaires.

Un mode de réalisation particulier de l'invention réside en l'utilisation d'un mélange racémique comprenant les composés cis-décaline (Ia) et (Ib) pour modifier ou renforcer les propriétés organoleptiques d'une substance, d'une composition ou d'un article.

On entend par «propriétés organoleptiques» toute propriété susceptible de modifier, améliorer ou renforcer la perception organoleptique d'une substance, d'une composition, d'un article par un utilisateur. Ainsi, à titre d'exemple préférentiel, l'agent organoleptique selon l'invention peut consister en un agent parfumant susceptible de conférer, modifier, améliorer ou renforcer la perception olfactive d'une substance, d'une composition ou d'un article.

Le principe général de l'invention repose sur la préparation et l'utilisation en parfumerie des composés de formule (I) décrits précédemment. Les exemples suivants illustrent une manière particulière de préparer les composés de l'invention, ainsi que le profil olfactif de chacun des composés exemplifiés. Ces exemples ne sont donnés que dans un but d'illustration et ne doivent pas être compris comme limitant la portée générale de l'invention.

### Exemple 1 Comparaison des évaluations olfactives du composé de formule (I) par rapport aux composés de l'art antérieur

| Composés divulgués ou composés de l'invention | Evaluation olfactive des composés divulgués dans le brevet EP 0029259 | Evaluation olfactive des composés divulgués dans le brevet US 4250338 | Evaluation olfactive par MANE des composés de l'art antérieur et de la présente invention |
|---|---|---|---|
| 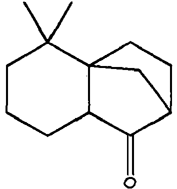 | Boisé, vert, menthe, camphré | Boisé, camphré, doux, avec des nuances menthées et ambrées | Aromatique, camphré, doux, légèrement terreux |
| 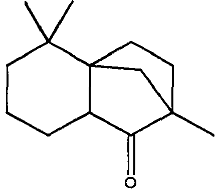 | Vert, camphré boisé | Boisé, avec nuances vertes, acides, cardamome | Camphré, boisé, nuances résineuses |
| 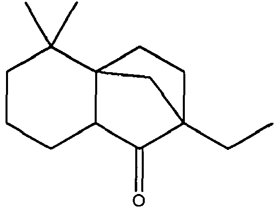 | | | Très faible, pratiquement sans odeur |
| 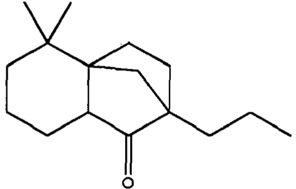 | | | Notes ambrées riches chaleureuses, propres, puissantes sans notes camphrées ou terreuses. Notes linéaires et très rémanentes |
| **Ia**/**Ib** | | | |
| **CIS**-**decaline configuration** | | | |
| 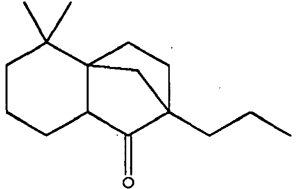 | | | Même évaluation olfactive que celle du composé cis-décaline mais intensité plus faible |
| **Ic / Id** | | | |
| **TRANS-decaline configuration** | | | |

### Exemple 2 Evaluation olfactive des composés cétoniques de l'art antérieur par rapport au composé selon l'invention

| Composés divulgués ou composés de l'invention | Evaluation olfactive des composés divulgués dans le brevet EP 0029259 | Evaluation olfactive des composés divulgués dans le brevet US 4250338 | Evaluation olfactive par MANE des composés .de l'art antérieur et de la présente invention |
|---|---|---|---|
| 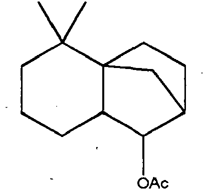 | ambré boisé | Boisé sec (cédre, vétiver), arômes type-ambrés et type santalés | |
| 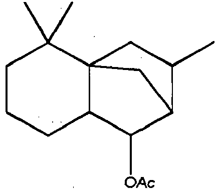 | ambré boisé | | |
| 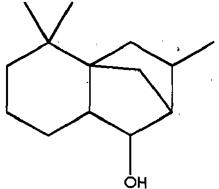 | | Boisé sec, type ambré, type castoreum, arôme cèdre, patchouli | |
| 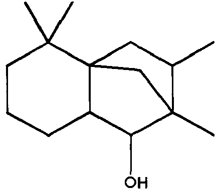 | | Boisé, ambré, arôme cèdre avec des nuances vertes et fruitées | |
| 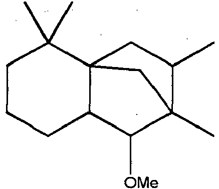 | | Boisé, ambré, type sandalwood, et arôme oriental | |
| 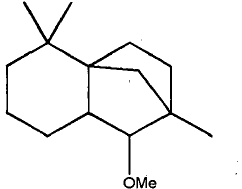 | | Ambré, boisé, camphré, profil aromatique doux-fruité | |
| 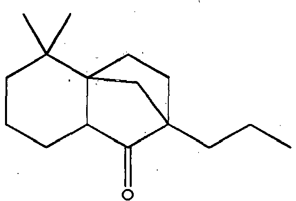 | | | Notes ambrées riches chaleureuses, propres, puissantes sans notes camphrées ou terreuses. Notes linéaires et très rémanentes |
| **Ia / Ib** | | | |
| **CIS-decaline configuration** | | | |
| 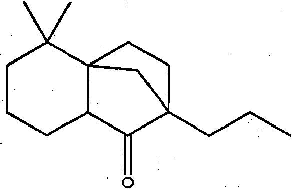 | | | Même évaluation olfactive que celle du composé cis-décaline mais intensité plus faible |
| **Ic / Id** | | | |
| **TRANS-decaline configuration** | | | |

### Exemple 3 : Synthèse d'un mélange racémique des composés (Ia) et (Ib)

Du myrcène (136g : 1 mol) est mis à réagir avec du 2-méthylènepentanal (120g : 1,22 mol) en présence d'AlCl₃ (8g) dans 350 ml de toluène, et ce pendant 16 heures à température ambiante. Après une distillation flash (point d'ébullition : 100 °C, 0.13 kPa (1 Torr)), on obtient 107 g d'un matériel qui est ensuite soumis à une cyclisation par un traitement à l'aide d'H₂SO₄ (4 g) dissous dans 600 ml de toluène, à 60 °C et pendant 24 heures. Après purification par distillation (point d'ébullition 104 °C, 0,8 Torr), on obtient 81 g d'un composé de formule (I). Si nécessaire, le composé de formule (I) peut être cristallisé à l'aide d'hexane à - 28 °C afin d'obtenir des cristaux ayant un point de fusion de 45 °C.
Analyse spectroscopique :
RMN ¹H (CDCl₃, 200 MHz) : δ 0,89 (t, 3H, J = 7 Hz) ; 0,95 (s, 3H) ; 0,99 (s, 3H) ; 1,17-1,93 (m, 16H) ; 2,00-2,06 (m, 1H).
RMN ¹³C (CDCl₃, 50 MHz) : δ 14, 96 (q) ; 19, 47 (t) ; 21, 02 (t) ; 21, 96 (t) ; 22, 54 (t) ; 23, 56 (q) ; 26, 28 (q) ; 31,55 (t) ; 32,68 (t) ; 33, 61 (s) ; 36, 77 (t) ; 41,05 (t) ; 51,97 (s) ; 53,84 (d) ; 59,37 (s) ; 220,72 (s).
IR : 1465, 1730, 2870, 2934, 2960 cm⁻¹
MS : 234 (61, M^{+.}), 205 (13), 191 (40), 177 (22), 165 (14), 164 (15), 163 (22), 151 (12), 150 (40), 149 (100), 135 (29), 126 (36), 122 (30), 121 (22), 109 (76), 108 (49), 107 (30), 105 (14), 95 (15), 93 (34), 91 (36), 81 (23), 79 (35), 77 (25), 69 (11), 67 (33), 55 (24), 41 (29).

### Exemple 4 Synthèse d'un mélange racémique de composés (Ic) et (Id)

Un mélange des composés (Ia) et (Ib) est mis en reflux dans du méthanol en présence de KOH (10 % w/w) pendant 24 heures jusqu'à complète isomérisation en composés (Ic) et (Id).
RMN 1H (CDCl3, 200 MHz) : δ 0,89 (m, 9H) ; 1, 12-1,85 (m, 16H) ; 1,89-2,00 (m, 1H).
RMN 13C (CDCl3, 50 MHz) : δ 14,96 (q) ; 19, 37 (t) ; 20,76 (t) ; 23,93 (q) ; 24,11 (t) ; 25,94 (q) ; 29,64 (t) ; 30,90 (t) ; 31,45 (t) ; 32,05 (s) ; 37,90 (t) ; 38,43 (t) ; 50,44 (s) ; 52,21 (d) ; 58,89 (s) ; 220,59 (s).
IR : 1462, 1739, 2869, 2929, 2955 cm-1.
MS : 234 (66, M+.), 205 (14), 191 (40), 177 (24), 165 (16), 164 (16), 163 (26), 151 (13), 150 (43), 149 (96), 135 (33), 126 (46), 125 (11), 123 (10), 122 (36), 121 (25), 110 (11), 109 (100), 108 (66), 107 (34), 105 (17), 95 (20), 93 (41), 91 (43), 81 (36), 80 (12), 79 (44), 77 (31), 69 (18), 67 (46), 65 (11), 55 (36), 53 (14), 43 (15), 41 (44), 39 (14).

### Exemple 5 : évaluation olfactive des composés (Ia) et (Ib) en mélange racémique

Les composés (Ia) et (Ib) ont été évalués dans une solution à 50 % de méthyl myristate : on constate une odeur ambrée, intense, et linéaire, qui dure très longtemps (plus de deux semaines).

### Exemple 6 Evaluation comparative d'une formule comprenant ou non le composé de formule (I)

| | **Composition 1** | **Composition 2** |
|---|---|---|
| **Matière Engagée** | **Parts (en poids)** | **Parts (en poids)** |
| Violettyne ¹⁾ | 10 | 10 |
| **Composé de formule (I)** ²⁾ | / | 60 |
| Calon 1951 Cal ³⁾ | 10 | 10 |
| Veramoss ⁴⁾ | 4 | 4 |
| Ethyl linalol ⁵⁾ | 100 | 100 |
| Florol ⁶⁾ | 20 | 20 |
| Dihydro jasmonate de méthyle ⁷⁾ | 574 | 574 |
| Helional ⁸⁾ | 60 | 60 |
| Iso E Super ⁹⁾ | 40 | 40 |
| Lilial ¹⁰⁾ | 20 | 20 |
| Melonal ¹¹⁾ | 2 | 2 |
| Methylionanthème ¹²⁾ | 10 | 10 |
| Salicylate de benzyle | 20 | 20 |
| Acétate de cis-3 hexényle ¹³⁾¹⁴⁾ | 4 | 4 |
| Acétate de styrallyle ¹⁴⁾ | 10 | 10 |
| Allyle amyle glycolate ¹⁴⁾ | 2 | 2 |
| Galbex ¹⁵⁾ | 4 | 4 |
| Cis-3 hexénol¹⁴⁾ | 4 | 4 |
| Liffarome ¹⁶⁾ | 4 | 4 |
| Triplal ¹⁴⁾¹⁷⁾ | 2 | 2 |
| DPG | 100 | 40 |
| Total | 1000 | 1000 |

| | | |
|---|---|---|
| 1. (E)-Undeca-1,3-dien-5-yne ; origine : Firmenich, Switzerland 2. Composé de formule (I) sous forme d'un mélange racémique; origine : V. Mane Fils, France. 3. 7-Methyl-benzo[b][1,4]dioxepin-3-one ; origine : Symrise, Allemagne. 4. 2,4-Dihydroxy-3,6-dimethyl-benzoic acid methyl ester ; origine : International Flavours and Fragrances, USA. 5. Origine : Givaudan, Switzerland. 6. 2-Isobutyl-4-methyl-tetrahydro-pyran-4-ol ; origine : Firmenich, Switzerland. 7. [3-oxo-2-((E)-pentyl)-cyclopentyl]-acetic acid methyl ester ; origine : Firmenich, Switzerland. 8. 3-Benzo[1,3]dioxol-5-yl-2-methyl-propionaldehyde ; origine : International Flavors and Fragrances, USA 9. 1-(2,3,8,8-Tetramethyl-1,2,3,4,5,6,7,8-octahydro-naphthalen-2-yl)-ethanone ; origine : International Flavours and Fragrances, USA. 10. 3-(4-tert-Butyl-phenyl)-2-methyl-propionaldehyde ; origine : Givaudan Switzerland. 11. 2,6-Dimethyl-hept-5-enal ; origine : Givaudan Switzerland. 12. (E)-3-Methyl-4-(2,6,6-trimethyl-cyclohex-2-enyl)-but-3-en-2-one ; origine : Firmenich, Switzerland. 13. Origine : V. Mane Fils, France. 14. 10 % dans dipropylène glycol. 15. Origine : Firmenich, Switzerland. 16. Carbonic acid (E)-hex-3-enyl ester methyl ester ; origine : International Flavors and Fragrances, USA. 17. 2,4-Dimethyl-cyclohex-3-enecarbaldehyde ; origine : International Flavors and Fragrances, USA. 18. Ethoxy-méthoxy-cyclododécane origine : Henkel, Allemagne. | | |

### Evaluation de la composition 2 / composition 1 :

Tête : puissante note ambrée-boisée dès de début de l'évaporation. Renforce les notes fruitées et vertes de la composition.

Coeur et fond : accord boisé et ambré élégant, s'associe très bien avec l'odeur marine de la composition créant un caractère unique et puissant. Longue rémanence sur mouillette.

## Revendications

1. Composé de formule générale (I) suivante :

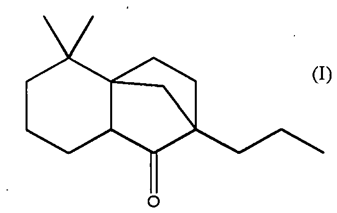

2. Composé selon la revendication 1, **caractérisé en ce que** le groupement décaline est dans une configuration cis (Ia) ou (Ib).

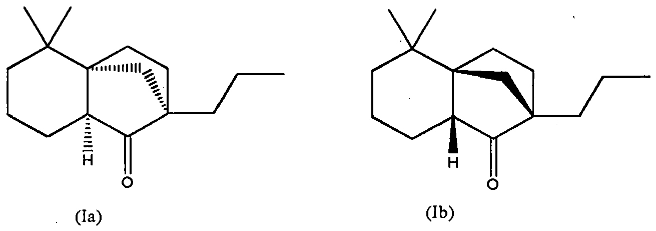

3. Composé selon la revendication 1, **caractérisé en ce que** le groupement décaline est dans une configuration trans (Ic) ou (Id).

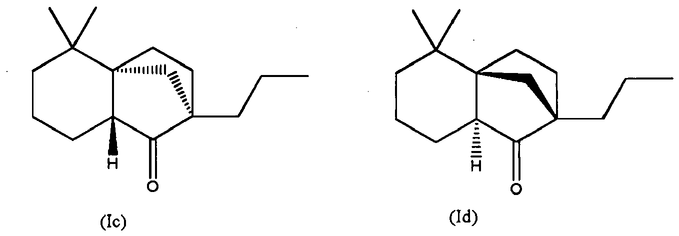

4. Composition **caractérisée en ce qu'**elle comprend au moins un composé de formule générale (I), (Ia), (Ib), (Ic) ou (Id) tel que défini dans l'une des revendications 1 à 3, sous forme d'un stéréoisomère ou d'un mélange de stéréoisomères, ou d'un mélange racémique.

5. Composition selon la revendication précédente **caractérisée en ce qu'**elle comprend en outre au moins une autre substance odorante.

6. Composition selon l'une des revendications 4 ou 5 **caractérisé en ce que** le composé est présent dans une concentration comprise entre 0.1 et 99 % en poids par rapport au poids total de la composition, notamment entre 0,1 et 30 %.

7. Utilisation d'au moins un composé de formule (I) tel que défini dans l'une des revendications 1 à 3 en tant qu'agent ou composé fragrant.

8. Utilisation d'au moins un composé de formule (I) tel que défini dans l'une des revendications 1 à 3 en tant qu'agent masquant d'une odeur ou neutralisant d'une odeur.

9. Utilisation, selon l'une des revendications 7 ou 8, d'au moins un composé de formule (I) seul ou en combinaison avec au moins un autre ingrédient aromatisant ou parfumant, et/ou au moins un solvant, et/ou au moins un adjuvant.

10. Utilisation selon l'une des revendications 7 à 9 pour conférer, modifier ou renforcer les propriétés organoleptiques d'une substance, d'une composition ou d'un article.

11. Utilisation selon l'une des revendications 7 à 10 d'un mélange racémique comprenant les composés cis-décaline (Ia) et (Ib) pour modifier ou renforcer les propriétés organoleptiques d'une substance, d'une composition ou d'un article.

## Patentansprüche

1. Eine Verbindung der folgenden allgemeinen Formel (I):

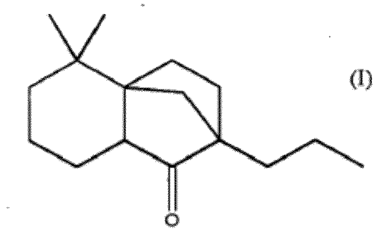

2. Verbindung gemäß Anspruch 1, **dadurch gekennzeichnet, dass** die Dekalingruppe in einer cis-Konfiguration (la) oder (Ib) ist.

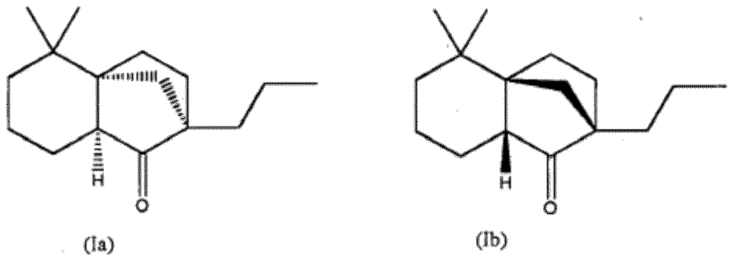

3. Verbindung gemäß Anspruch 1, **dadurch gekennzeichnet, dass** die Dekalingruppe in einer trans-Konfiguration (Ic) oder (Id) ist.

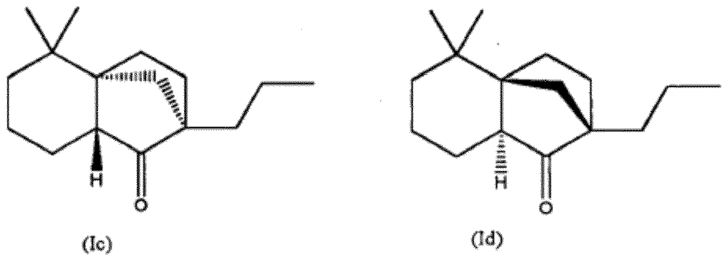

4. Eine Zusammensetzung, **dadurch gekennzeichnet, dass** sie mindestens eine Verbindung der allgemeinen Formel (I), (la), (Ib), (Ic) oder (Id), wie in einem der Ansprüche 1 bis 3 definiert, in Form eines Stereoisomers oder einer Mischung aus Stereoisomeren oder einer racemischen Mischung beinhaltet.

5. Zusammensetzung gemäß dem vorhergehenden Anspruch, **dadurch gekennzeichnet, dass** sie ferner mindestens einen anderen Geruchstoff beinhaltet.

6. Zusammensetzung gemäß einem der Ansprüche 4 oder 5, **dadurch gekennzeichnet, dass** die Verbindung in einer Konzentration zwischen 0,1 und 99 Gew.-%, bezogen auf das Gesamtgewicht der Zusammensetzung, insbesondere zwischen 0,1 und 30 %, vorliegt.

7. Eine Verwendung mindestens einer Verbindung der Formel (I), wie in einem der Ansprüche 1 bis 3 definiert, als Duftmittel oder Duftverbindung.

8. Eine Verwendung mindestens einer Verbindung der Formel (I), wie in einem der Ansprüche 1 bis 3 definiert, als Geruchsmaskierungsmittel oder Geruchsneutralisierungsmittel.

9. Verwendung gemäß einem der Ansprüche 7 oder 8 mindestens einer Verbindung der Formel (I) alleine oder in Kombination mit mindestens einem anderen Aromainhaltsstoff oder Parfüminhaltsstoff und/oder mindestens einem Lösungsmittel und/oder mindestens einem Zusatzmittel.

10. Verwendung gemäß einem der Ansprüche 7 bis 9 zum Vermitteln, Modifizieren oder Verstärken der organoleptischen Eigenschaften eines Stoffes, einer Zusammensetzung oder eines Artikels.

11. Verwendung gemäß einem der Ansprüche 7 bis 10 einer racemischen Mischung, beinhaltend die Zusammensetzungen cis-Dekalin (la) und (Ib) zum Modifizieren oder Verstärken der organoleptischen Eigenschaften eines Stoffes, einer Zusammensetzung oder eines Artikels.

## Claims

1. A compound of the following general formula (I):

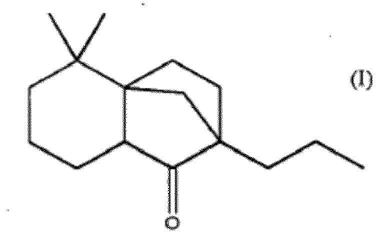

2. A compound according to Claim 1, **characterised in that** the decalin group is in a cis configuration (Ia) or (Ib).

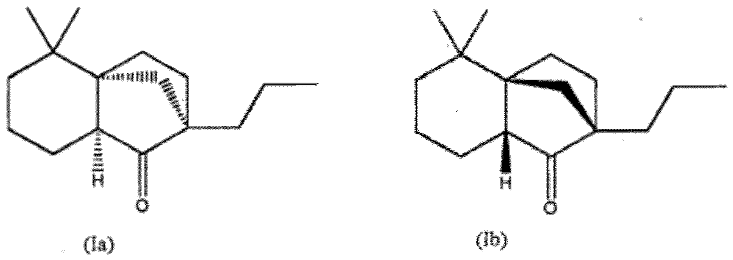

3. A compound according to Claim 1, **characterised in that** the decalin group is in a trans configuration (Ic) or (Id).

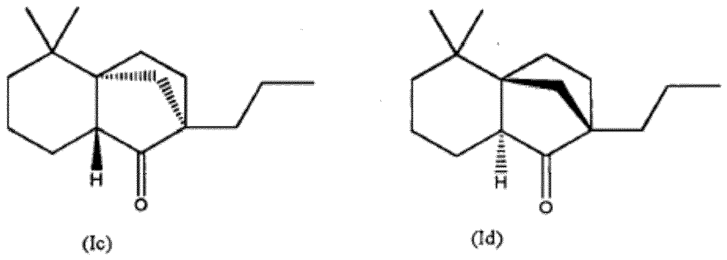

4. A composition **characterised in that** it comprises at least one compound of general formula (I), (Ia), (Ib), (Ic) or (Id) such as defined in one of claims 1 to 3, in the form of a stereoisomer or a mixture of stereoisomers, or a racemic mixture.

5. The composition according to the preceding claim, **characterised in that** it further comprises at least one other odorising substance.

6. The composition according to one of claims 4 or 5, **characterised in that** the compound is present in a concentration of between 0.1 and 99% by weight relative to the total weight of the composition, particularly between 0.1 and 30%.

7. The use of at least one compound of formula (I) such as defined in one of claims 1 to 3 as a fragrant agent or compound.

8. The use of at least one compound of formula (I) such as defined in one of claims 1 to 3 as an odour-masking or odour-neutralising agent.

9. The use, according to one of claims 7 or 8, of at least one compound of formula (I) alone or in combination with at least one other aromatic or perfuming ingredient, and/or at least one solvent, and/or at least one additive.

10. The use according to one of claims 7 to 9 to confer, modify or reinforce the organoleptic properties of a substance, a composition, or an article.

11. The use according to one of claims 7 to 10 of a racemic mixture comprising the cis-decalin compounds (Ia) and (Ib) to modify or reinforce the organoleptic properties of a substance, a composition, or an article.
